# EUROPEAN PATENT APPLICATION

(11) **EP 4 793 600 A1**
(43) Date of publication of application: **19.08.2026**
(21) Application number: 25157661.7
(22) Date of filing: 13.02.2025
(51) Int. Cl.: G01D 11/24

(54) **SENSOR AND METHOD FOR MANUFACTURING THE SAME**

(71) Applicant: Pepperl+Fuchs SE, 68307 Mannheim (DE)
(72) Inventor: Speiger, Marco, 68307 Mannheim (DE)
(74) Representative: Tergau & Walkenhorst Intellectual Property GmbH

(57) **Abstract**

A method for manufacturing a sensor (1), comprising:
- a step a) of providing a sensor housing (2), comprising sensor electronics (5);
- a step b) of inserting a negative geometry (9) into an opening of the sensor housing (2) intended for the installation of a sensor interface (3) and/or sensor interface functional element (3a, 3b, 4), wherein the negative geometry (9) represents the counter contour of the sensor interface (3) and/or the sensor interface functional element (3a, 3b, 4);
- a step c) of potting the sensor electronics (5) of the sensor (1) with a casting compound (8);
- a step d) of curing the casting compound (8);
- a step e) of removing the negative geometry (9), whereby a contour (14) is formed in the cured casting compound (8) that maps the geometry of the sensor interface (3) and/or sensor interface functional element (3a, 3b, 4).

## Description

### FIELD OF THE INVENTION

The present invention relates to a method for manufacturing a sensor, and a sensor.

### BACKGROUND OF THE INVENTION

Sensors are critical components in various applications, including industrial automation, automotive systems, and consumer electronics. The performance and reliability of these sensors are highly dependent on the precision and stability of their internal components, particularly the sensor electronics and sensor interface functional elements.

Traditional methods of manufacturing sensors often involve complex assembly processes that can lead to misalignment and instability of the sensor interface functional elements. These issues can result in reduced sensor accuracy and reliability, which are critical parameters in high-performance applications. Additionally, the conventional methods may require extensive manual labor and intricate tooling, increasing production costs and time.

In the state of the art, challenges are the cost-efficient production, however, also the precise alignment and secure attachment of sensor interface functional elements within the sensor housing.

Particularly, in relation to small sensor sizes, the narrow cross-sections create difficulties in accommodating components, for example sensor electronics, as well as challenges during assembly or potting processes. Therefore, there is an unmet need for additional space within the sensor to address these issues. Also achieving precise alignment of cylindrical sensor housings is challenging, leading to larger tolerances and consequently lower quality results. When using an internal housing, the contact surface of the potting compound with the sleeve is reduced, resulting in loose sensor electronics.

### SUMMARY OF THE INVENTION

It is thus an objective of the present invention to provide a method for manufacturing sensors that ensures precise alignment and secure attachment of sensor interface functional elements within the sensor housing.

It is a further object of the present invention to simplify the assembly process of sensors.

It is a still further objective of the present invention to enhance the stability and reliability of the sensor components.

It is a still further objective of the present invention to provide a versatile manufacturing method that can accommodate various types of sensor interface functional elements and sensor designs.

These and other problems are solved by the subject matter of the attached independent claims.

The present invention addresses these objectives by introducing a novel method for manufacturing sensors that ensures precise alignment and secure attachment of sensor interface functional elements within the sensor housing. This method involves the use of a negative geometry to create accurate contours in a casting compound, which are then used to securely hold the sensor interface functional elements. By incorporating the negative geometry technique, the method further reduces the need for complex tooling and extensive manual labor. By simplifying the assembly process and enhancing the stability of the sensor components, this invention aims to improve the overall performance and reliability of sensors while reducing manufacturing complexity and cost.

Particularly, the present invention offers several advantages. Due to space constraints with the internal housing, the connection pins partially covered by the sleeve must be soldered onto the PCB. By using the "negative geometry" concept underlying the present invention, the assembly can be pre-assembled and then inserted. The positioning of the grounding wire can be placed under the HMI when using such negative geometry. In the prior art with the internal housing, the foil must be cut out for this purpose, which adds extra effort in positioning the foil and can impair functionality. Additionally, there is a risk of damaging the grounding wire during assembly with the internal housing. The narrow gaps with the internal housing prevent the potting process from being completed in one step, which increases process time and the risk of air inclusions. The additional space gained by the present invention, thus advantageously can be used at least partially for designing HMI control elements, improving both assembly and durability.

### DETAILED DESCRIPTION OF THE INVENTION

The above objects of the invention are achieved by a method for manufacturing a sensor according to a first aspect of the present invention and a sensor according to a second aspect of the present invention.

Preferred embodiments may be taken from the dependent claims, and, beyond that, from the following description, in particular comprising various embodiments as covered and described in the annexed claims.

The embodiments, features and combination of features as described herein in connection with the invention, as well as the combination of features as given in the annexed claims, but also any combination of features as mentioned and described in connection with the embodiments shall be considered as being disclosed herein, at least, however, shall be considered to be derivable by the skilled person.

Further, each feature and each combination of features in the claims and used in the description below may be used and claimed independently from the respective claimed subject matter, independently from claim dependencies and back-references, and independently from the claim category in which the feature is claimed. For example, in an arbitrary combination selected from one or more claims, one or more embodiments as set forth herein below and/or from the annexed figures may be envisaged.

The above-described problems are advantageously solved by a method for manufacturing according to a first aspect of the present invention. Said method comprises at least the following steps:
- a step a) of providing a sensor housing, comprising sensor electronics;
- a step b) of inserting a negative geometry into an opening of the sensor housing intended for the installation of a sensor interface and/or sensor interface functional element, wherein the negative geometry represents the counter contour of the sensor interface and/or the sensor interface functional element;
- a step c) of potting the sensor electronics of the sensor with a casting compound;
- a step d) of curing the casting compound;
- a step e) of removing the negative geometry, whereby a contour is formed in the cured casting compound that maps the geometry of the sensor interface and/or sensor interface functional element.

The term "sensor housing", as used herein preferably refers to a structure designed to accommodate or encompass sensor electronics. A sensor housing may also be provided in the form of a sensor sleeve. A sensor housing may be provided either as a single-piece or a multi-piece construction, providing flexibility in design and assembly. The sensor housing or sensor sleeve ensures the protection and stability of the internal sensor components.

The term "negative geometry," as used herein, preferably refers to a form or shape, preferably a solid form or shape, that represents the counter contour of an intended sensor interface functional element. This negative geometry is inserted into an opening of the sensor housing during the manufacturing process. It serves as a mold or template to create precise contours in a casting compound, capable of holding the sensor interface functional elements securely in place. By using negative geometry, the method ensures accurate alignment and secure attachment of the sensor interface functional elements within the sensor housing.

In an advantageous embodiment, the casting compound may be translucent, which advantageously allows for the transmission of light, enabling to include a viewing window for a status indicator.

The negative geometry may have various forms suitable to practice the present invention. For example, the negative geometry may be provided in the form of a plug. Such plug is inserted into an opening of the sensor housing during the manufacturing process.

The term "sensor interface" as used herein, preferably refers to the component or system that facilitates communication between a sensor and other devices or systems or a user, enabling the sensor to transmit data and/or to receive commands. The sensor interface may include various types of sensor interface functional elements, such as structural sensor interface functional elements, like frame elements, connectors, covers, or other attachment features; and/or the sensor interface may comprise further sensor interface functional elements, such as sensor interface display elements, such as a light guide or 7-segment display or the like, to display information to a user; and/or the sensor interface may comprise further sensor interface functional elements, such as sensor interface setting elements, such as rotary knobs, push buttons, and other similar controls, that enable to adjust or input settings of the sensor. Particularly the sensor interface may enable the sensor to communicate with or be integrated into different environments. Particularly the sensor interface may be in the form of a human-machine interface, allowing the setting of the sensor by a user and/or displaying information to the user.

The term "sensor interface functional element" as used herein, preferably refers to a specific part or feature of the sensor interface that performs a distinct function, such as signal processing, data conversion, or user interaction. For example, light guide or a display or a turn element or a rotary actuator or a turn element or a frame to hold said elements can be considered sensor interface functional elements. The light guide might be used to direct light to a sensor, while the rotary actuator or turn element could be used to adjust settings or input data. These elements may all together form the sensor interface.

The term "casting compound," as used herein, preferably refers to a material used to encapsulate and protect the sensor electronics within the sensor housing. This compound is typically a liquid or semi-liquid substance that hardens or cures over time to form a solid, protective layer. The casting compound ensures the stability and durability of the sensor components by providing mechanical support and shielding them from environmental factors such as moisture, dust, and mechanical stress. Preferably, the casting compound is a potting resin, which is well-suited for creating a robust and reliable encapsulation for the sensor electronics. For example, an epoxy casting resin can be used, optionally with suitable mineral fillers or the like.

The term "mapping of the geometry," as used herein, refers to the process of creating a contour in the cured casting compound that corresponds to at least a portion of the contact surface of the sensor interface functional element. This mapping ensures that the formed contour accurately reflects the necessary parts of the sensor interface functional element's geometry required for secure attachment and alignment, rather than replicating the entire contour of the entire element. This precise mapping is crucial for maintaining the stability and functionality of the sensor by ensuring that the sensor interface functional elements are properly positioned and held in place.

The term "removing," as used herein, preferably refers to the process of extracting or eliminating the negative geometry from the cured casting compound. The removal process ensures that the sensor interface and/or the sensor interface functional element, respectively, can be securely and precisely positioned within the sensor housing.

It shall be noted that the steps given above do not necessarily have to be carried out in the given order. The provided steps may be carried out in any other suitable order.

However, the order as given above may apply for particular variants of the method. For example, removing the negative geometry, may be necessarily performed after curing the casting compound.

The above-described problems are also advantageously solved by a sensor according to the second aspect of the present invention. Such sensor comprises a sensor interface and/or a sensor interface functional element held by a contour formed by cured casting compound.

The sensor according to the present invention, thereby preferably is manufactured according to the method of the first aspect of the invention. A skilled person can immediately distinguish sensors manufactured using the inventive method due to the unique characteristics of the casting compound. For example, when the sensor is cut open, the specific contours and features formed in the cured casting compound are clearly visible. These contours, which map at least a portion of the contact surface of the sensor interface functional elements, are distinctive and indicative of the inventive manufacturing process. This visibility allows for a straightforward identification of sensors produced by this method, as the precise and secure attachment of the sensor interface functional elements within the sensor housing is evident from the appearance of the casting compound.

Within the present application, terms such as "lateral" or "laterally", "rear", "frontal", "upper", "lower", "bottom", "opposite", "inner", "outer" or the like, as used herein, which describe the position of a first object relative to another object, preferably refer to the relative position of a respective part or object with regard to its position fully mounted for its intended use.

It will be immediately acknowledged by a person skilled in the art that a feature, embodiment, effect or advantage described herein in connection with the inventive sensor, may also be a feature, embodiment, effect or advantage of the inventive method, respectively, and vice versa.

In an advantageous embodiment of the inventive method, particularly in a step f) a first sensor interface functional element of the sensor interface, preferably a structural sensor sensor interface functional element, is inserted into the contour of the cured casting compound.

This is of advantage in that it ensures the precise and secure placement of the first sensor interface functional element within the sensor housing. The contour formed in the casting compound is specifically designed to match at least a portion of the contact surface of the sensor interface functional element, providing a stable and accurate fit.

In a further advantageous embodiment of the inventive method, particularly in a step g), at least one further sensor interface functional element of the sensor interface, preferably a sensor interface display element (3a) or a sensor interface actuation element, particularly a turn element (3b), is inserted into the contour of the cured casting compound and/or into the first sensor interface functional element of the sensor interface.

The advantage of this embodiment extends the invention's applicability to multiple sensor interface functional elements. Particularly, this embodiment allows for a modular approach to sensor design. By enabling the insertion of additional sensor interface functional elements into the existing contour or into the first sensor interface functional element, the sensor can be easily customized or upgraded. This modularity provides flexibility in adapting the sensor to different applications or requirements without needing a complete redesign. Furthermore, inserting further sensor interface functional elements into the contour of the cured casting compound or into the first sensor interface functional element ensures a secure and precise fit. This integration enhances the stability and reliability of the sensor, as all components are firmly held in place, reducing the risk of misalignment or dislodgement.

In a further advantageous embodiment of the inventive method, particularly in a step e), and/or of the inventive sensor forming a contour in the casting compound comprises forming at least one undercut.

This is advantageous in that incorporating an undercut significantly enhances the secure attachment of the sensor interface functional elements within the sensor housing. The undercut provides a mechanical interlock that prevents the sensor interface functional elements from shifting or becoming dislodged, even under mechanical stress or vibration. This ensures that the sensor interface functional elements remain precisely aligned and securely held in place, which is crucial for maintaining the sensor's accuracy and reliability.

In other words, having contours with undercuts means that the sensor interface functional elements can be more robustly integrated into the sensor housing. The undercuts allow for the use of fastening elements, such as snap hooks, which can engage with the undercuts to provide a detachable or non-detachable connection. In this regard, the sensor interface functional element may comprise a fastening element provided to engage with an undercut of the contour.

More particularly, if a counter contour with elastic properties is used, such as an elastic silicone plug, due to the elastic properties and the non-stick effect of the surface, the silicone plug can be easily removed from the cured casting resin despite the undercut, allowing for multiple uses and thus reducing costs. A corresponding injection-molded part would be difficult or impossible to demold due to the required undercut, which would increase complexity and make the part expensive

In a further advantageous embodiment of the inventive sensor and/or of the inventive method, particularly a step f) of inserting a first sensor interface functional element into the contour and/or step g) of inserting at least one further sensor interface functional element into the at least one first sensor interface functional element, the first sensor interface functional element and/or the at least one further sensor interface functional element, respectively, is connected with the contour.

Such insertion may be achieved in various ways, known to the person skilled in the art. Preferably, the insertion comprises the engagement of the respective sensor interface functional element with an undercut. Particularly the sensor interface functional element comprises a respective fastening element configured to engage with at least one undercut. Specifically, the sensor interface functional element can include snap hooks as fastening elements that securely lock into the undercut. This engagement ensures that the sensor interface functional elements are held firmly in place, preventing any movement or dislodgement. When the sensor interface functional element is pressed into place, it engages, preferably with a fastening element, with the undercut, thereby effectively locking the sensor interface functional elements and preventing them from falling out.

Accordingly, in a further advantageous embodiment of the inventive method and/or the inventive sensor connecting the first sensor interface functional element with the contour and/or the at least one further sensor interface functional element with the contour comprises fastening a at least one fastening element, particularly snapping at least one snap hook element, of the sensor interface into the undercut of the first contour.

In a further advantageous embodiment of the inventive method, particularly step e), and/or of the inventive sensor removing the negative geometry comprises melting or disintegrating the negative geometry.

This is advantageous, in that it allows for a clean and precise removal of the negative geometry without causing any damage or disturbance to the cured casting compound. This method ensures that the contours formed in the casting compound remain intact and accurately reflect the intended geometry of the sensor interface functional elements. Particularly, a negative geometry in the form of a plug, offers a straightforward and efficient approach. The plug can be easily inserted into the sensor housing and, after the casting compound has cured, it can be simply pulled out and reused. This reuse of the plug reduces material costs and simplifies the manufacturing process.

In a further advantageous embodiment of the inventive method and/or the inventive sensor, the first sensor interface functional element is a sealing element and wherein step f) of inserting a first sensor interface functional element into the contour of the cured casting compound comprises a step of inserting the sealing element into the contour.

Such sealing element advantageously provides an additional barrier against environmental factors such as moisture, dust, and contaminants. This protection is crucial for maintaining the integrity and performance of the sensor in harsh or variable conditions.

More importantly, however, the sealing element as such may form the contour, and thus the negative geometry represents the counter contour of the sealing element of the sensor interface functional element.

In a further advantageous embodiment of the inventive method and/or the inventive sensor the contour comprises at least one undercut designed to receive a fastening element of the sensor interface functional element.

This is particularly advantageous, as the inclusion of at least one undercut in the contour significantly enhances the secure attachment of the sensor interface functional element within the sensor housing. The undercut may be specifically designed to receive a fastening element of the sensor interface functional element, such as a snap hook or similar locking mechanism. This design ensures that the sensor interface functional element is firmly held in place, preventing any movement or dislodgement during operation.

In a further advantageous embodiment of the inventive method and/or the inventive sensor the sensor interface functional element comprises at least one fastening element, preferably a snap element, in particular a snap hook, designed to connect the sensor interface functional element with the undercut of the contour.

The use of snap hooks as fastening elements simplifies the assembly process, allowing for quick and easy insertion of the sensor interface functional elements into the sensor housing. This reduces the need for additional tools or complex assembly procedures, thereby streamlining production and reducing manufacturing costs. Moreover, the snap hook design allows for both detachable and non-detachable connections, offering flexibility in the assembly and maintenance of the sensor.

In a further advantageous embodiment of the inventive method and/or the inventive sensor the undercut is designed to at least partially surround an opening of the sensor housing intended to receive the sensor interface functional element.

This is particularly advantageous, as designing the undercut to at least partially surround an opening of the sensor housing intended to receive the sensor interface functional element significantly enhances the secure attachment and stability of the sensor interface functional element. By partially encircling the opening, the undercut provides a robust mechanical interlock that ensures the sensor interface functional element is firmly held in place. This design minimizes the risk of the sensor interface functional element becoming dislodged or misaligned during operation, which is crucial for maintaining the sensor's accuracy and reliability.

In a further advantageous embodiment of the inventive method and/or the inventive sensor the sensor interface functional element comprises a first sensor interface functional element and at least one further sensor interface functional element, wherein preferably the first sensor interface functional element is a sealing element, and wherein more preferably the sealing element is made of an elastomer.

This configuration provides enhanced sealing capabilities, ensuring that the sensor remains protected from environmental factors such as moisture and dust. The use of an elastomer for the sealing element offers superior flexibility and durability, which contributes to the longevity and reliability of the sensor. Additionally, the presence of multiple sensor interface functional elements allows for a more secure and stable connection, further improving the overall performance of the sensor.

In a further advantageous embodiment of the inventive method and/or the inventive sensor the first sensor interface functional element, particularly in the form of an sensor interface functional element sealing, comprises at least one fastening element designed to connect the sensor interface functional element sealing with the undercut of the contour, and wherein most preferably the at least one further sensor interface functional element is held by the first sensor interface functional element.

This design ensures a secure and stable connection between the sealing element and the sensor housing, enhancing the overall integrity of the sensor. Moreover, the configuration allows for at least one further sensor interface functional element to be held by the first sensor interface functional element. This arrangement not only simplifies the assembly process but also improves the sealing efficiency. The presence of the fastening element ensures that the sealing element is firmly anchored in place, while the additional further sensor interface functional element contributes to a tighter seal, thereby providing superior protection against environmental factors such as moisture and dust.

In a further advantageous embodiment at least two further sensor interface functional element are inserted into the sealing element, for example a light guide and a rotary actuator, turn element, or the like, which are to be held by the first sensor interface functional element, i.e. the sealing element. This arrangement not only simplifies the assembly process but also improves the sealing efficiency. The presence of the fastening element ensures that the sealing element is firmly anchored in place, while the additional sensor interface functional elements contribute to a tighter seal.

In a further advantageous embodiment of the inventive method and/or the inventive sensor the sensor interface functional element is configured such that the further sensor interface functional element is held by the interface sealing element and presses the sensor interface functional element sealing, in particular a fastening element of the interface sealing element, if present, into or against the undercut.

This configuration offers several benefits. Firstly, it ensures a more secure and stable connection between the sensor interface functional elements and the sensor housing. By pressing the sealing element into the undercut, the further sensor interface functional element enhances the sealing effect, providing superior protection against environmental factors such as moisture and dust. This not only improves the overall durability and reliability of the sensor but also simplifies the assembly process. The additional pressure exerted by the further sensor interface functional element ensures that the sealing element remains firmly in place, reducing the risk of dislodgement or leakage. Consequently, this embodiment significantly enhances the performance and longevity of the sensor, making it more robust and effective for various applications.

All described embodiments of the invention have the advantage of ensuring precise alignment and secure attachment of sensor interface functional elements within the sensor housing. This precision enhances the overall performance and reliability of the sensors, which is critical for high-performance applications.

The sensor according to the present invention advantageously can be used for detecting sensor signals, preferably in the manufacturing of components, mechanical and plant engineering, automotive manufacturing, mobile equipment, storage and conveyor technology, packaging industry, and in factory and process automation. The sensor can be any sensor, for example preferably a capacitive sensor, making it highly suitable for these applications due to its sensitivity and accuracy.

Particularly, by using the counter contour, e.g. a plug, instead of the HMI housing solves several problems and offers the following advantages:

By using the counter contour, the ring cross-section of the HMI housing is eliminated, which increases the effective cross-section for potting, thereby simplifying the manufacturing process. This reduces the process time for potting and simultaneously improves quality by reducing air bubbles in the potting compound, which primarily form in narrow channels.

The positioning of the HMI housing requires a device that is complex and tolerance-prone. Additionally, there is a risk of incorrectly inserting the asymmetrical HMI housing. By using the counter contour, this device is no longer necessary, and the position of the HMI contour is reproducible due to the play-free fit of the plug in the sleeve.

When inserting the HMI housing into the sleeve, the sealing lips of the HMI housing can be sheared off at the window of the sleeve. When passing the sealing lip of the HMI housing, an additional step is required in the inner geometry of the sleeve. Furthermore, a high surface quality is necessary for the sealing function. Using the plug and the negative geometry according to the present invention, thus reduces the corresponding machining costs of the sleeve.

Eliminating the housing wall thickness between the HMI and electronics creates more space for the HMI components.

For the sensor to function without interference, grounding is required, which involves welding a grounding braid to the sleeve. The present invention has the advantage that, without the HMI housing, the entire space can be utilized to position the grounding weld, which was previously extremely tight. This increases the effort in manufacturing the assembly device for positioning the foil. Using the plug allows the grounding braid to be freely positioned.

Particularly, in the case of a cylindrical design, an undercut can be created as described herein on the inner contour of the sleeve, which prevents the HMI cover from falling out. In the production of (flat) plastic housings, creating such an undercut is very complex due to the injection molding process. Specific tools, which can be sensible to damages are usually needed.

### BRIEF DESCRIPTION OF THE FIGURES

The present invention will be described in further detail with reference to the drawings from which further features, embodiments and advantages may be taken, and in which:
Fig. 1A shows a top view of a cylindrical sensor according to a first embodiment of the present invention;
Fig. 1B shows a schematic cross-section of a cylindrical sensor according to the prior art;
Fig. 2A and Fig. 2B illustrate cross-sections of the cylindrical sensor of the present invention according to the first inventive embodiment, depicting two representative steps in the inventive manufacturing method;
Fig. 2C illustrates an enlarged detail view of Fig. 2B;
Fig. 3A illustrates a schematical cross-sectional view of a rectangular sensor of the prior art;
Fig. 3B illustrates a schematical cross-sectional view of the rectangular sensor interface according to a second inventive embodiment;
Fig. 4A and 4B illustrates a schematical cross-section of a third inventive embodiment from two different perspectives.

The features of the present invention disclosed in the specification, the claims, and/or the figures may both separately and in any combination thereof be material for realizing the invention in various forms thereof.

In the embodiments shown in the figures, elements similar or identic in function are designated with like reference signs. It is noted that the figures may not be true to scale with respect to each other.

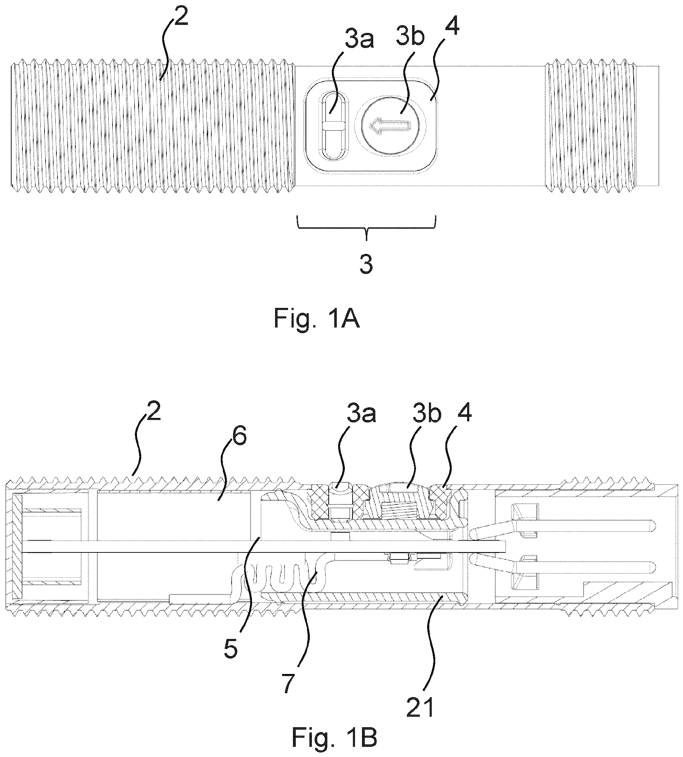

FIG 1A shows a schematic top view of the sensor according to the present invention. The sensor comprises a sensor housing 2, which encloses the sensor components and provides structural support. The sensor interface 3 is positioned within the housing and serves as the primary connection point for external elements and the actuation or setting by the user. The first sensor interface functional element 4, which functions as a sealing element is shown integrated into the sensor interface 3 to ensure a secure and tight seal. Additionally, the figure depicts further sensor interface functional elements 3a and 3b include displaying and operating elements, respectively, such as a light guide 3a and a turn element 3b, connected to the sensor interface 3.

FIG 1B illustrates a schematic cross-section of a sensor according to the prior art. The sensor housing 2 contains the sensor electronics 5. The sensor interface 3 is integrated within the housing and serves as the connection point for external elements. The first sensor interface functional element 4, which functions as a sealing element, is positioned to ensure a secure seal within the sensor interface 3. The further sensor interface functional elements 3a and 3b, which can be any suitable displaying or operating element, respectively, such as a light guide 3a or a turn element 3b, are also shown connected to the sensor interface 3. In the prior art, the HMI housing 21 is a separate component that must be individually manufactured, inserted, and assembled. This additional HMI housing 21 encloses the human-machine interface components 3a, 3b and 4 of the sensor interface 3. The sensor electronics 5 are protected by a foil 6 and comprise a grounding braid 7 to ensure proper electrical grounding. The sensor electronics 5 are accommodated within the sensor housing 2, providing additional protection and stability. The sensor interface 3 is allocated with in an opening of the sensor housing 2. This prior art configuration highlights the complexity and the need for multiple separate manufacturing and assembly steps, particularly for the HMI housing 21, which must be handled independently.

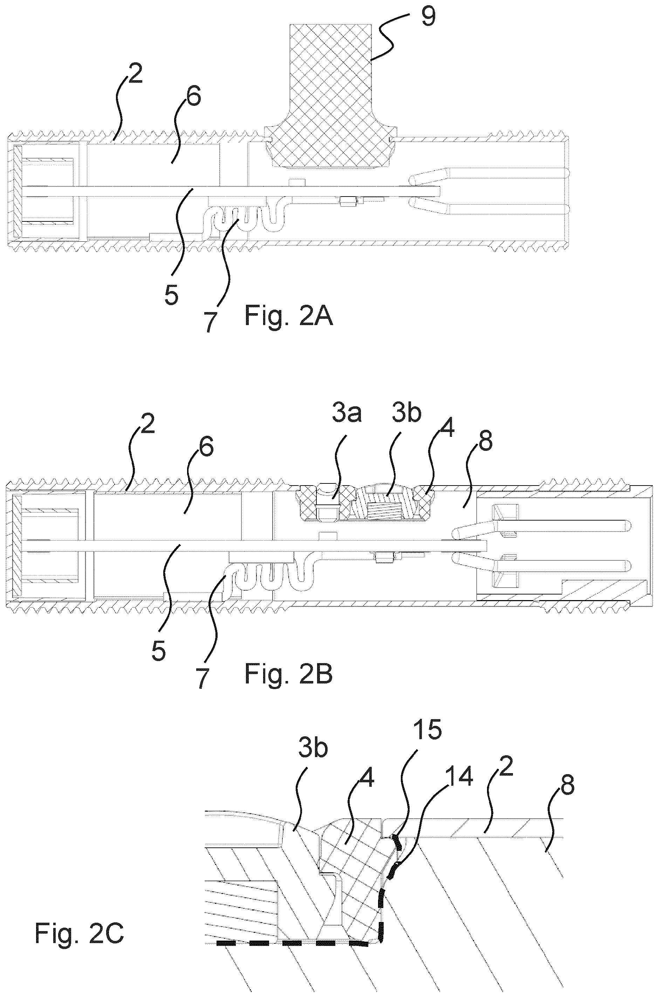

FIG. 2A, FIG 2B and 2C illustrate cross-sections of the sensor of the present invention according to the first inventive embodiment, depicting two representative steps in the inventive manufacturing method in FIG 2A and 2B. More particularly, FIG 2A shows the sensor after a step b) of inserting a negative geometry 9 into an opening of the sensor housing 2. The negative geometry 9 is depicted as a plug, which represents the counter contour of the sensor interface 3. This step is crucial for shaping the final form of the sensor interface. Particularly, according to the inventive method, for manufacturing the sensor in a step a) a sensor housing 2 is provided, which comprises sensor electronics 5. Then in the step b) the negative geometry 9 is inserted into the opening of the sensor housing 2, which is intended for the installation of the sensor interface 3. Thereby, the negative geometry 9 represents the counter contour 14 of the sensor interface 3 as shown particularly in Fig. 2C by the dotted line.

With the plug 9 inserted in the respective position in a step c) the sensor electronics 5 of the sensor 1 are potted with a casting compound 8, which is cured in a step d) of the present inventive method.

Figure 2B illustrates the result of step e) after the negative geometry 9 has been removed, revealing the opening with the contour 14 (shown as a dotted line in FIG 2C) formed in the cured casting compound 8. This contour 14 maps the geometry of the sensor interface 3, and particularly the sensor interface functional element 4. FIG 2B also shows the results of subsequent steps f) and g), where the first sensor interface functional element 4, in the form of a sealing element, is inserted into the contour 14, and subsequently, the further sensor interface functional elements 3a and 3b, here displaying and operating elements in the form of a light guide 3a and an turn element 3b, are inserted into the first sensor interface functional element 4, i.e. the sealing element.

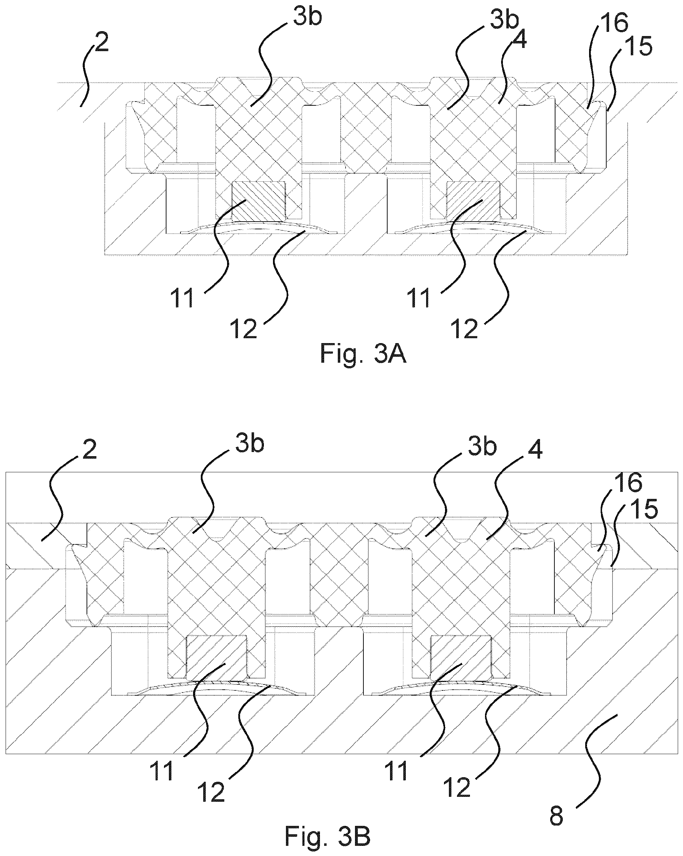

FIGs 3A provides a detailed cross-sectional views of the sensor interface 3 according to a further embodiment of the prior art and FIG: 3B shows a respective inventive embodiment of the present inventive sensor, here a rectangular sensor.

Fig. 3A and Fig 3B show the sensor interface 3, which includes a first sensor interface functional element 4, in the form of a sealing element 4 and two further sensor interface functional elements 3b, here in the form of two adjacent turn elements.

Fig. 3A shows the sensor interface 3, where the housing 2 is made as a casting mold according to the prior art. Thereby, the shown undercut 15 is difficult to manufacture, which is specifically designed to receive the fastening elements 16, here in the form of snap-hooks 16 of the sealing element 4 of the sensor interface functional element 4. This undercut 15 provides a secure anchoring point, ensuring that the sensor interface functional element 3 is firmly held in place.

Figure 3B further details the present invention. Here the casting material 8, is as a result of step e) after the negative geometry 9 has been removed, revealing the opening with the contour 14 (according to what is shown as a dotted line in FIG 2C) formed in the cured casting compound 8. This contour 14 of the casting compound 8 maps the geometry of the sensor interface 3, and particularly the sensor interface functional element 4. FIG 3B also shows the results of subsequent steps f) and g), where the first sensor interface functional element 4, in the form of a sealing element, is inserted into the contour 14, and subsequently, the further sensor interface functional elements 3b, here operating elements in the form of an turn element 3b, are inserted into the first sensor interface functional element 4, i.e. the sealing element.

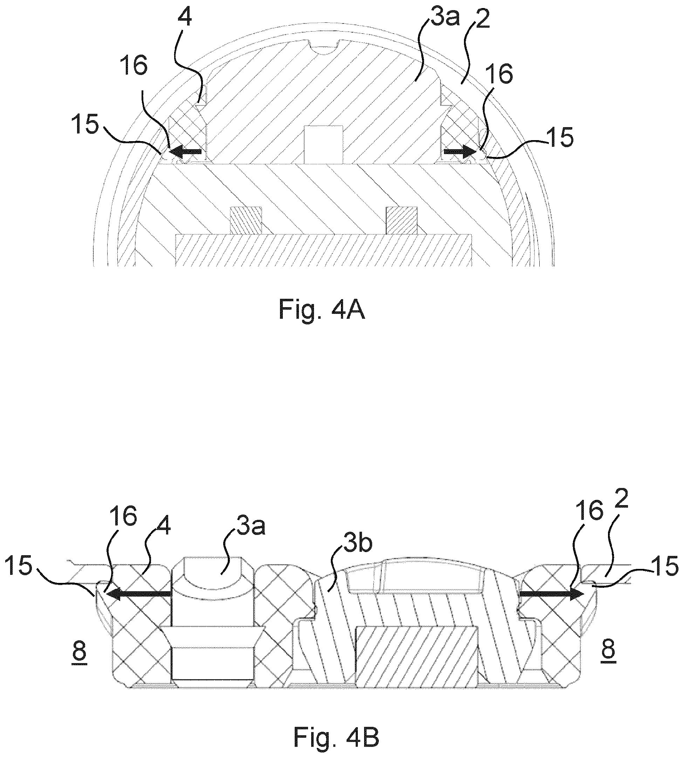

This particular mechanism is best seen in FIGs 4A and 4B. These figures illustrate the detailed process of how the snap hook element 16 is pushed into the undercut 15 of the contour 14 formed by the cured casting compound 8.

Figure 4A shows the initial state where the first further sensor interface functional element 3a is inserted into the first sensor interface functional element 4, which is a sealing element. The sealing element 4 is already positioned within the contour 14 of the cured casting compound 8. Figure 4A depicts the insertion of a second further sensor interface functional element 3b into the first sensor interface functional element 4. As this second further sensor interface functional element 3b is inserted, it exerts a force (F) on the first sensor interface functional element 4, specifically on the snap hook element 16. This force (depicted by the arrows in Fig. 4A and 4B) pushes the snap hook element 16 into the undercut 15 of the contour 14 (which can be best seen in Fig. 4A and 4B, for example), ensuring a secure and stable connection. Figure 4A shows the final state where both further sensor interface functional elements 3a and 3b are fully inserted in the first sensor interface functional element 4, and which form the sensor interface 3. The force exerted by the second further sensor interface functional element 3b has firmly pressed the snap hook element 16 into the undercut 15, enhancing the sealing effect and stability of the connection. This mechanism ensures that the sensor interface functional elements 3a, 3b and 4, all are securely held in place, providing superior protection against environmental factors and improving the overall reliability and performance of the sensor.

The embodiments in the figures may relate to preferred embodiments, while all elements and features described in connection with embodiments may be used, as far as appropriate, in combination with any other embodiment and feature as discussed herein, in particular related to any other embodiment discussed further above.

### LIST OF REFERENCE NUMERALS

- 2: sensor housing
- 21: HMI housing
- 3: Sensor interface
- 3a, 3b: further sensor interface functional element, operating element
- 4: first sensor interface functional element, sealing
- 5: sensor electronics
- 6: foil
- 7: grounding braid
- 8: casting compound
- 9: negative geometry, plug
- 11: magnet
- 12: snap disc
- 14: contour
- 15: undercut
- 16: fastening element, snap hook element

## Claims

1. A method for manufacturing a sensor (1), comprising:
- a step a) of providing a sensor housing (2), comprising sensor electronics (5);
- a step b) of inserting a negative geometry (9) into an opening of the sensor housing (2) intended for the installation of a sensor interface (3) and/or sensor interface functional element (3a, 3b, 4), wherein the negative geometry (9) represents the counter contour of the sensor interface (3) and/or the sensor interface functional element (3a, 3b, 4);
- a step c) of potting the sensor electronics (5) of the sensor (1) with a casting compound (8);
- a step d) of curing the casting compound (8);
- a step e) of removing the negative geometry (9), whereby a contour (14) is formed in the cured casting compound (8) that maps the geometry of the sensor interface (3) and/or sensor interface functional element (3a, 3b, 4).

2. The method according to claim 1, further comprising a step f) of inserting a first sensor interface functional element (4), preferably a structural sensor interface functional element (4), of the sensor interface (3) into the contour (14) of the cured casting compound (8).

3. The method according to any one of claims 1 or 2, further comprising a step g) of inserting at least one further sensor interface functional element (3a, 3b) of the sensor interface (3), preferably a sensor interface display element (3a) or a sensor interface setting element (3b), into the contour (14) of the cured casting compound (8) and/or into the first sensor interface functional element (4) of the sensor interface.

4. The method according to any one of claims 1 to 3, wherein step e) of forming the contour (14) in the casting compound (8) comprises forming of at least one undercut (15)

5. The method according to any one of claims 1 to 4, preferably 4, wherein step f) of inserting a first sensor interface functional element (4) into the contour (14) and/or step g) of inserting at least one further sensor interface functional element (3a, 3b) into the at least one first sensor interface functional element (4) comprises connecting the first sensor interface functional element (4) and/or the at least one further sensor interface functional element (3a, 3b), respectively, with the contour (14).

6. The method according to claim 5, wherein connecting the first sensor interface functional element (4) with the contour (14) and/or the at least one further sensor interface functional element (31) with the contour (14) comprises fastening a at least one fastening element (16), particularly snapping at least one snap hook element (16) of the sensor interface and/or sensor interface functional element (3) into the undercut (15) of the first contour (14).

7. The method according to any one of claims 1 to 6, wherein step e) of removing the negative geometry (9) comprises melting or disintegrating the negative geometry (9).

8. The method according to any one of claims 2 to 7, wherein the first sensor interface functional element (4) is a sealing element (4) and wherein step f) of inserting a first sensor interface functional element (4) into the contour (14) of the cured casting compound (8) comprises a step of inserting the sealing element (4) into the contour (14).

9. A sensor (1), preferably manufactured according to the method of any one of claims 1 to 8, comprising a sensor interface and/or sensor interface functional element (3) held by a contour (14) formed by cured casting compound (8).

10. The sensor (1) according to claim 9, wherein the contour (14) comprises at least one undercut (15) designed to receive a fastening element (16) of the sensor interface (3).

11. The sensor (1) according to any one of claims 9 or 10, wherein the sensor interface functional element (3) comprises at least one fastening element (16), preferably a snap element, in particular a snap hook, designed to connect the sensor interface functional element (3) with the undercut (15) of the contour (14).

12. The sensor (1) according to any one of claims 9 to 11, wherein the undercut (15) is designed to at least partially surround an opening of the sensor housing (2) intended to receive the sensor interface (3) and/or the sensor interface functional element (3a, 3b, 4).

13. The sensor (1) according to any one of claims 8 to 12, wherein the sensor interface (3) comprises at least one first sensor interface functional element (4) and at least one further sensor interface functional element (3a, 3b), wherein preferably the first sensor interface functional element (4) is a sealing element (4), and wherein more preferably the first sensor interface functional element (4) is made of an elastomer.

14. The sensor (1) according to any one of claims 8 to 13, wherein the first sensor interface functional element (4), particularly in the form of an sensor interface functional element sealing (4), comprises at least one fastening element (16) designed to connect the sensor interface functional element sealing (4) with the undercut (15) of the contour (14), and wherein most preferably the at least one further sensor interface functional element (3a, 3b) is held by the first sensor interface functional element (4).

15. The sensor (1) according to any one of claims 8 to 14, wherein the further sensor interface functional element (3a, 3b) is configured such that the further sensor interface functional element (3a, 3b) is held by the interface sealing element (4) and presses the interface sealing element (4), in particular a fastening element (16) of the interface sealing element (4), if present, into or against the undercut (15).
